# EUROPEAN PATENT APPLICATION

(11) **EP 3 828 897 A1**
(43) Date of publication of application: **02.06.2021**
(21) Application number: 21165224.3
(22) Date of filing: 26.03.2021
(51) Int. Cl.: G16H 50/50

(54) **METHOD AND APPARATUS FOR PREDICTING RESULT OF APPEARANCE CHANGING OPERATION, DEVICE, STORAGE MEDIUM AND COMPUTER PROGRAM PRODUCT**

(30) Priority: 30.06.2020 CN 202010608567
(71) Applicant: Beijing Baidu Netcom Science and Technology Co., Ltd., Beijing 100085 (CN)
(72) Inventor: DONG, Ziyu, Beijing, Beijing 100085 (CN)
(74) Representative: Müller, Christian Stefan Gerd

(57) **Abstract**

The present disclosure discloses a method and apparatus for predicting a result of an appearance changing operation, and relates to the field of artificial intelligence, big data, and image processing technology. The method includes: acquiring sample data of the appearance changing operation; establishing a result predicting model of the appearance changing operation based on the sample data; and acquiring appearance data and an intended appearance changing operation of a target user, to predict appearance data of the target user after the intended appearance changing operation using the result predicting model of the appearance changing operation based on the appearance data and the intended appearance changing operation of the target user. This method can be used to provide the user with predicted appearance data after the appearance changing operation, to assist the user in making decisions.

## Description

### TECHNICAL FIELD

Embodiments of the present disclosure relate to the field of artificial intelligence technology, specifically to big data technology and image processing technology, and more specifically to a method and apparatus for predicting a result of an appearance changing operation by modeling using a big data processing platform.

### BACKGROUND

With the improvement of people's living standards, people begin to pay attention to their appearance, and wish to predict their appearance changes with, e.g., age and lifestyle, by technical means. Some people further wish to change their appearance by adopting physical exercises or a lifestyle of healthy diets, or by, e.g., a medical beauty surgery. Before considering changing their appearance by selecting a lifestyle or a medical beauty surgery, people mainly consider unpredictable results.

Therefore, a method for predicting a result of an appearance changing operation is required.

### SUMMARY

Embodiments of the present disclosure provide a method and apparatus for predicting a result of an appearance changing operation, an electronic device, a computer readable storage medium, and a computer program product.

In a first aspect, an embodiment of the present disclosure provides a method for predicting a result of an appearance changing operation, the method including: acquiring sample data of the appearance changing operation, the sample data comprising first appearance data of a user before the appearance changing operation and second appearance data after the appearance changing operation corresponding to the first appearance data; establishing a result predicting model of the appearance changing operation based on the first appearance data and the second appearance data; and acquiring appearance data and an intended appearance changing operation of a target user, to predict appearance data of the target user after the intended appearance changing operation using the result predicting model of the appearance changing operation based on the appearance data and the intended appearance changing operation of the target user.

In a second aspect, an embodiment of the present disclosure provides an apparatus for predicting a result of an appearance changing operation, the apparatus including: an acquiring unit configured to acquire sample data of the appearance changing operation, the sample data comprising first appearance data of a user before the appearance changing operation and second appearance data after the appearance changing operation corresponding to the first appearance data; a modeling unit configured to establish a result predicting model of the appearance changing operation based on the first appearance data and the second appearance data; and a predicting unit configured to acquire appearance data and an intended appearance changing operation of a target user, to predict appearance data of the target user after the intended appearance changing operation using the result predicting model of the appearance changing operation based on the appearance data and the intended appearance changing operation of the target user.

In a third aspect, an embodiment of the present disclosure provides an electronic device, the device electronic including: one or more processors; and a storage apparatus for storing one or more programs, the one or more programs, when executed by the one or more processors, causing the one or more processors to perform the method for predicting a result of an appearance changing operation according to the first aspect.

In a fourth aspect, an embodiment of the present disclosure provides a computer readable storage medium storing a computer program thereon, the program, when executed by a processor, causing the processor to perform the method for predicting a result of an appearance changing operation according to the first aspect.

In a fifth aspect, an embodiment of the present disclosure provides a computer program product including a computer program, where the computer program, when executed by a processor, implements the method for predicting a result of an appearance changing operation according to the first aspect.

The method and apparatus for predicting an appearance change provided in embodiments of the present disclosure first establish a result predicting model based on sample data of an appearance changing operation, and then predict appearance data of a target user after the appearance changing operation based on the result predicting model, thereby providing the user with predicted appearance data after the appearance changing operation to assist the user in making decisions.

It should be understood that the content described in this section is neither intended to identify key or important features of embodiments of the present disclosure, nor intended to limit the scope of the present disclosure. Other features of the present disclosure will become readily understood in conjunction with the following description.

### BRIEF DESCRIPTION OF THE DRAWINGS

The accompanying drawings are used for better understanding of the present solution, and do not impose a limitation on the present disclosure.
Fig. 1 is a diagram of an example system architectural in which embodiments of the present disclosure may be implemented;
Fig. 2 is a flowchart of a method for predicting a result of an appearance changing operation according to an embodiment of the present disclosure;
Fig. 3 is a schematic diagram of an application scenario of the method for predicting a result of an appearance changing operation according to an embodiment of the present disclosure;
Fig. 4 is a flowchart of the method for predicting a result of an appearance changing operation according to another embodiment of the present disclosure;
Fig. 5 is a flowchart of the method for predicting a result of an appearance changing operation according to still another embodiment of the present disclosure;
Fig. 6 is a schematic structural diagram of an apparatus for predicting a result of an appearance changing operation according to an embodiment of the present disclosure; and
Fig. 7 is a block diagram of an electronic device for implementing the method for predicting a result of an appearance changing operation of embodiments of the present disclosure.

### DETAILED DESCRIPTION OF EMBODIMENTS

Example embodiments of the present disclosure are described below with reference to the accompanying drawings, including various details of the embodiments of the present disclosure to contribute to understanding, which should be considered merely as examples. Therefore, those of ordinary skills in the art should realize that various alterations and modifications can be made to the embodiments described here without departing from the scope and spirit of the present disclosure. Similarly, for clearness and conciseness, descriptions of well-known functions and structures are omitted in the following description.

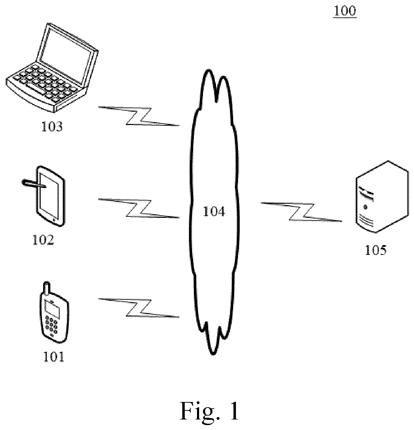

Fig. 1 shows an example system architecture 100 in which a method for predicting a result of an appearance changing operation or an apparatus for predicting a result of an appearance changing operation of embodiments of the present disclosure may be implemented.

As shown in Fig. 1, the system architecture 100 may include terminal devices 101, 102, and 103, a network 104, and a server 105. The network 104 serves as a medium providing a communication link between the terminal devices 101, 102, and 103, and the server 105. The network 104 may include various types of connections, such as wired or wireless communication links, or optical fiber cables.

A user may interact with the server 105 using the terminal devices 101, 102, and 103 via the network 104, e.g., to receive or send a message. The terminal devices 101, 102, and 103 may be provided with various client applications receiving pushed services, e.g., an image application, and a data collection application.

The terminal devices 101, 102, and 103 may be various electronic devices having a display screen and supporting receiving the pushed services, including but not limited to a smart phone, a tablet computer, an e-book reader, an MP3 (Moving Picture Experts Group Audio Layer III) player, an MP4 (Moving Picture Experts Group Audio Layer IV) player, a laptop portable computer, a desktop computer, and the like.

The terminal devices 101, 102, and 103 may be hardware, or may be software. When the terminal devices 101, 102, and 103 are hardware, the terminal devices may be various electronic devices, while when the terminal devices 101, 102, and 103 are software, the terminal devices may be installed in the above-listed electronic devices, may be implemented as a plurality of software programs or software modules (e.g., a plurality of software modules configured to provide distributed services) , or may be implemented as a single software program or software module. This is not specifically limited here.

The server 105 can classify or filter appearance change data using a technology in the big data field to acquire sample data of appearance changes, then model the appearance changes based on the sample data and using a technology in the artificial intelligence field, acquire appearance data and a type of the appearance changing operation of a user from the terminal devices 101, 102, and 103, predict the appearance changes of the user using an appearance change predicting model, and provide a predicting result to the terminal devices 101, 102, and 103.

It should be noted that the method for predicting a result of an appearance changing operation provided in embodiments of the present disclosure is generally executed by the server 105. Accordingly, the apparatus for predicting a result of an appearance changing operation is generally provided in the server 105.

It should be understood that numbers of terminal devices, networks, and servers in Fig. 1 are merely illustrative. Any number of terminal devices, networks, and servers may be provided based on actual requirements.

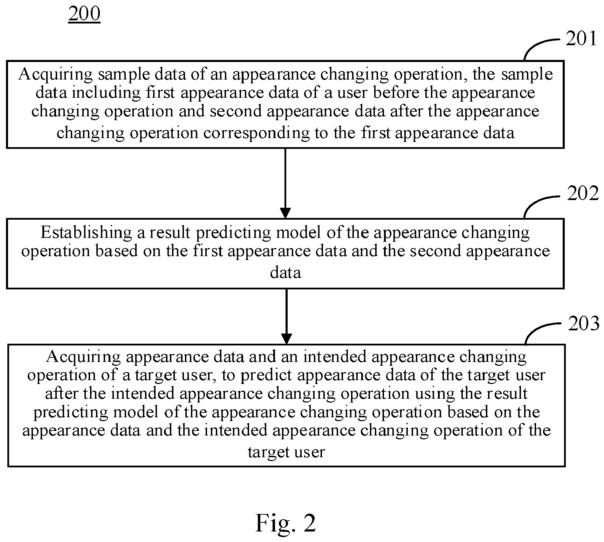

Further referring to Fig. 2, a process 200 of a method for predicting a result of an appearance changing operation according to an embodiment of the present disclosure is shown. The method for predicting a result of an appearance changing operation, includes the following steps.

Step 201: acquiring sample data of an appearance changing operation, the sample data including first appearance data of a user before the appearance changing operation and second appearance data after the appearance changing operation corresponding to the first appearance data.

In the present embodiment, an executing body (e.g., the server shown in Fig. 1) of the method for predicting a result of an appearance changing operation may acquire sample data of the appearance changing operation from a terminal or the Internet, and the sample data includes the first appearance data, i.e., the appearance data of the user before the appearance changing operation, and the second appearance data, i.e., the appearance data of the user after the appearance changing operation corresponding to the appearance data of the user before the appearance changing operation. The appearance data may be various kinds of data characterizing appearance features of the user, e.g., a facial image, or a three-dimensional facial model based on dense key points.

As an example, the sample data may be facial images tracking appearance changes of the user in 10 years. The appearance changing operation is an appearance change of the user with age, the appearance data of the user before the appearance changing operation may be facial images of the user in a 1st year of sample collection, the appearance data of the user after the appearance changing operation may be facial images of the user in a 10th year of sample collection, and the corresponding appearance changing operation is an operation of "increasing age. " Alternatively, the sample data may be case data of a medical beauty surgery, the appearance changing operation may be, e.g., a medical beauty surgery or a beautifying operation, or an operation for a specified part of a human body in the medical beauty surgery, e.g., a double eyelid operation, the appearance data of the user before the appearance changing operation may be facial images of the user before the medical beauty surgery, and the appearance data of the user after the appearance changing operation may be facial images of the user after the medical beauty surgery.

Step 202: establishing a result predicting model of the appearance changing operation based on the first appearance data and the second appearance data.

In the present embodiment, the result predicting model of the appearance changing operation is established using a data mining technology, regression analysis, a neural network, or other algorithms, based on the appearance data of the user before the appearance changing operation, and the appearance data of the user after the appearance changing operation corresponding to the appearance data of the user before the appearance changing operation among the sample data. The result predicting model of the appearance changing operation is used for predicting the appearance data after the appearance changing operation based on the appearance data before the appearance changing operation. Modeling may be performed by plurality of approaches. For example, establishing the result predicting model using a neural network algorithm may include first performing iterative training on an initial training model based on the appearance data of the user before the appearance changing operation and the appearance data of the user after the appearance changing operation in the sample data, generating feedback information using a difference between the appearance data of the user before the appearance changing operation and the appearance data of the user after the appearance changing operation (e.g., a difference between feature values of preset features), updating the initial model, stopping training when the number of iterations of training meets a preset number of iterations, and using the initial model that meets the number of iterations of training as a final result predicting model. Alternatively, modeling may be performed based on a mathematical model, e.g., a linear regression model, and parameters in the model may be fitted based on the sample data.

Step 203: acquiring appearance data and an intended appearance changing operation of a target user, to predict appearance data of the target user after the intended appearance changing operation using the result predicting model of the appearance changing operation based on the appearance data and the intended appearance changing operation of the target user.

In the present embodiment, the appearance data of the target user before the appearance changing operation and the intended appearance changing operation of the target user are acquired based on user inputs or by reading data sent by the user, and the appearance data of the target user before the appearance changing operation and the intended appearance changing operation of the target user are inputted into the result predicting model of the appearance changing operation, to predict the appearance data of the target user after the intended appearance changing operation using the result predicting model of the appearance changing operation.

In some application scenarios, it is also possible to evaluate a success rate of an appearance operation of the target user based on the predicted appearance data of the target user after the appearance changing operation. For example, when the appearance changing operation is a medical beauty surgery, facial images of the target user before the medical beauty surgery and previous case data may be analyzed to analyze operation success rates corresponding to preoperative facial images in the case data having similar features or proportions with preoperative facial images of the target user, and an operation success rate of the target user may be determined based on operation success rates of similar users in the case data.

The method for predicting an appearance change provided in the present embodiment first establishes a result predicting model based on sample data of an appearance changing operation, and then predicts appearance data of a target user after the appearance changing operation based on the result predicting model, thereby providing the user with predicted appearance data after the appearance changing operation to assist the user in making decisions.

Alternatively, the establishing the result predicting model of the appearance changing operation based on the first appearance data and the second appearance data includes: performing key point feature extraction on the first appearance data and the second appearance data; and modeling a conversion relationship for converting a key point feature of the first appearance data to a key point feature of the second appearance data, to obtain the result predicting model.

In the present embodiment, key points of the appearance data may be preset, a key point feature of the user before change may be extracted from the appearance data before the appearance changing operation, a key point feature of the user after change may be extracted from the appearance data of the user after the appearance changing operation, and modeling may be performed based on the conversion relationship for converting the key point feature before change to the key point feature after change, to obtain the result predicting model. The key point feature may be a feature that has a great impact on appearance and characterizes an appearance feature, such as a size of an eye or a shape of a nose. Specifically, modeling may be performed using a relation modeling algorithm, e.g., a neural network algorithm, regression analysis, or recursive analysis, based on the conversion relationship for converting the key point feature before change to the key point feature after change, to obtain the result predicting model. In the present embodiment, key points are selected, and the result predicting model is established based on changes of the key point feature, thereby establishing the result predicting model with accurate predicting results in the case of using less data, and improving the modeling efficiency.

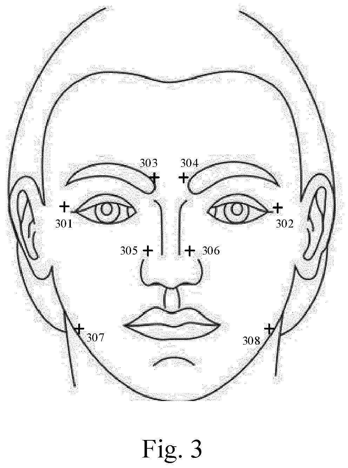

In some application scenarios, as shown in Fig. 3, points 301 to 308 in a preset facial image are key points of the facial image (it is understandable that the key points may be any point in the facial image, and the key points selected here are used merely as examples) . Feature values of the key point 301 to the key point 308 (e.g., reflectance degrees of the key points, an unevenness degrees of the key points, or a gap or a positional relationship between the key points) of a user before an appearance changing operation may be collected using a facial image scanning system for use as first appearance data, feature values of the key point 301 to the key point 308 of the user after the appearance changing operation may be collected for use as second appearance data, and a result predicting model of the appearance changing operation may be established based on the first appearance data and the second appearance data. After a target user inputs his facial image before the appearance changing operation into the result predicting model of the appearance changing operation, and selects an intended appearance changing operation, the result predicting model may compute feature values of the key point 301 to the key point 308 of the facial image of the target user after the appearance changing operation based on feature values of the key point 301 to the key point 308 of the target user before the appearance changing operation, and output a facial image of the target user after the appearance changing operation generated based on the feature values of the key point 301 to the key point 308 of the facial image of the target user after the appearance changing operation.

Alternatively, the sample data further includes physical state data of the user corresponding to the second appearance data, and the establishing the result predicting model of the appearance changing operation based on the first appearance data and the second appearance data includes: establishing the result predicting model of the appearance changing operation based on the first appearance data and the corresponding second appearance data, and the physical state data of the user.

In the present embodiment, the result predicting model of the appearance changing operation is established using a data mining technology, regression analysis, or other algorithms, based on the appearance data of the user before the appearance changing operation, the appearance data of the user after the appearance changing operation corresponding to the appearance data of the user before the appearance changing operation, and the physical state data of the user corresponding to the appearance data of the user after the appearance changing operation in the sample data. The physical state data of the user may be a physical health condition, e.g., a sequelae symptom from which the user suffers after a medical beauty surgery. In the present embodiment, the physical state data of the user corresponding to the appearance data of the user after the appearance changing operation is used as one of the factors for establishing the result predicting model, and other physical states except for appearance changes after the appearance changing operation may also be used as a predicting result, thereby providing the user with multi-dimensional predicting result information.

Alternatively, the sample data further includes the physical state data of the user corresponding to the second appearance data, and the method for predicting a result of an appearance changing operation further includes: predicting physical state information of the target user after the intended appearance changing operation using the result predicting model of the appearance changing operation.

In the present embodiment, the physical state information of the target user after the appearance changing operation can be predicted using the result predicting model based on a type of the appearance changing operation selected by the target user (e.g., a surgical item of the medical beauty surgery), such that the target user knows about the physical state information after the appearance operation change, thereby providing the user with multi-dimensional predicting result information, and helping the user to make accurate decisions.

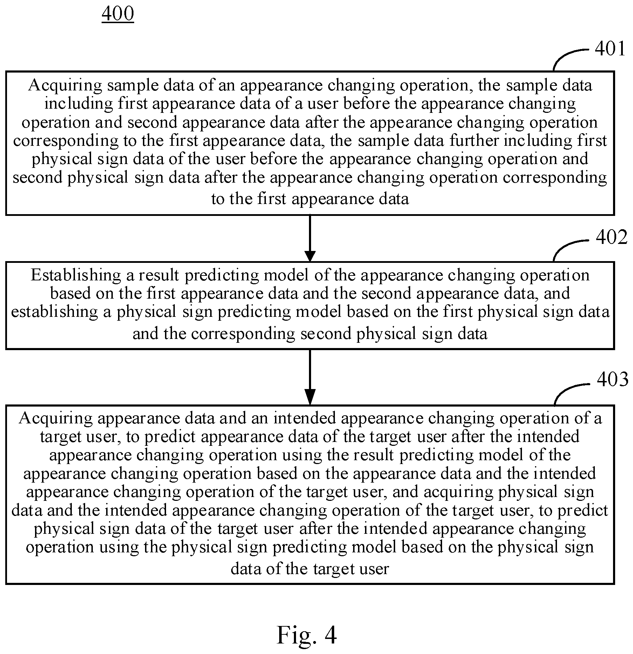

Further referring to Fig. 4, a process 400 of the method for predicting a result of an appearance changing operation according to another embodiment is shown, including the following steps.

Step 401: acquiring sample data of an appearance changing operation, the sample data including first appearance data of a user before the appearance changing operation and second appearance data after the appearance changing operation corresponding to the first appearance data, the sample data further including first physical sign data of the user before the appearance changing operation and second physical sign data after the appearance changing operation corresponding to the first appearance data.

Description of the first appearance data and the second appearance data in the present embodiment is consistent with the description in step 201. The description will not be repeated here.

In the present embodiment, the sample data of the appearance changing operation may be acquired from a terminal or the Internet, and the sample data includes the first physical sign data, i.e., the physical sign data of the user before the appearance changing operation, and the second physical sign data, i.e., the physical sign data of the user after the appearance changing operation corresponding to the physical sign data of the user before the appearance changing operation. For example, the sample data may be case data of a medical beauty surgery, the appearance changing operation may be, e.g., a medical beauty surgery or a beautifying operation, the physical sign data of the user before the appearance changing operation may be a physical health condition of the user before the medical beauty surgery, and the physical sign data of the user after the appearance changing operation may be a physical health condition of the user after the medical beauty surgery, e.g. , whether a sequela arises.

Step 402: establishing a result predicting model of the appearance changing operation based on the first appearance data and the second appearance data, and establishing a physical sign predicting model based on the first physical sign data and the corresponding second physical sign data.

Description of the result predicting model in the present embodiment is consistent with the description of step 202. The description will not be repeated here.

In the present embodiment, the physical sign predicting model is established using a data mining technology, regression analysis, or other algorithms, based on the physical sign data of the user before the appearance changing operation, and the physical sign data of the user after the appearance changing operation corresponding to the physical sign data of the user before the appearance changing operation among the sample data. The physical sign predicting model is used for predicting the physical sign data after the appearance changing operation based on the physical sign data before the appearance changing operation.

Step 403: acquiring appearance data and an intended appearance changing operation of a target user, to predict appearance data of the target user after the intended appearance changing operation using the result predicting model of the appearance changing operation based on the appearance data and the intended appearance changing operation of the target user, and acquiring physical sign data and the intended appearance changing operation of the target user, to predict physical sign data of the target user after the intended appearance changing operation using the physical sign predicting model based on the physical sign data of the target user.

Description of the predicting the appearance data of the target user after the appearance changing operation using the result predicting model based on the appearance data of the target user before the appearance changing operation in the present embodiment is consistent with the description of step 203. The description will not be repeated here.

In the present embodiment, the physical sign data of the target user before the appearance changing operation and the intended appearance changing operation of the target user are acquired based on user inputs or by reading data sent by the user, and the physical sign data of the target user before the appearance changing operation is inputted into the physical sign predicting model, to predict the physical sign data of the target user after the intended appearance changing operation using the physical sign predicting model.

The method for predicting an appearance change provided in the present embodiment first establishes a physical sign predicting model based on sample data of an appearance changing operation, and then predicts physical sign data of a target user after the appearance changing operation based on the physical sign predicting model, thereby providing the user with predicted physical sign data after the appearance changing operation to assist the user in making decisions.

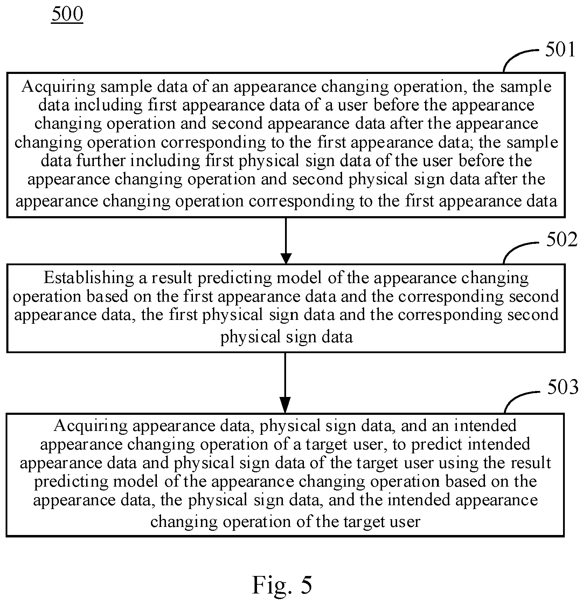

Further referring to Fig. 5, a process 500 of the method for predicting a result of an appearance changing operation according to still another embodiment is shown, including the following steps.

Step 501: acquiring sample data of an appearance changing operation, the sample data including first appearance data of a user before the appearance changing operation and second appearance data after the appearance changing operation corresponding to the first appearance data; the sample data further including first physical sign data of the user before the appearance changing operation and second physical sign data after the appearance changing operation corresponding to the first appearance data.

Description of the first appearance data, the second appearance data, the first physical sign data, and the second physical sign data in the present embodiment is consistent with the description in step 201 and step 401. The description will not be repeated here.

Step 502: establishing a result predicting model of the appearance changing operation based on the first appearance data and the corresponding second appearance data, the first physical sign data and the corresponding second physical sign data.

In the present embodiment, the result predicting model of the appearance changing operation is established using a data mining technology, regression analysis, or other algorithms, based on the appearance data of the user before the appearance changing operation, the appearance data of the user after the appearance changing operation corresponding to the appearance data of the user before the appearance changing operation, the physical sign data of the user before the appearance changing operation, and the physical sign data of the user after the appearance changing operation corresponding to the physical sign data of the user before the appearance changing operation in the sample data. In the present embodiment, the result predicting model is established based on multi-dimensional data before the appearance changing operation and multi-dimensional data after the appearance changing operation, thereby improving the prediction accuracy of the established result predicting model.

Step 503: acquiring appearance data, physical sign data, and an intended appearance changing operation of a target user, to predict intended appearance data and physical sign data of the target user using the result predicting model of the appearance changing operation based on the appearance data, the physical sign data, and the intended appearance changing operation of the target user.

In the present embodiment, the appearance data and the physical sign data of the target user after the intended appearance changing operation are predicted through the result predicting model based on the appearance data and the physical sign data of the target user before the appearance changing operation, and the intended appearance changing operation of the target user. For example, the appearance changing operation is a rhinoplasty surgery, and an image and a health condition of the nose of the target user after the rhinoplasty surgery, e.g., whether a sequelae, e.g., distortion or blockage of the bridge of the nose, will arise, may be determined based on a preoperative image and a preoperative health condition of the nose of the target user, e.g., whether the bridge of the nose has been cracked due to a heavy blow. In the present embodiment, the appearance data and the physical sign data of the target user after the appearance operation are predicted using the result predicting model established based on the appearance data and the physical sign data, such that the predicting model can perform multi-dimensional analysis on the data of the target user after the appearance changing operation, and then the predicting result is more accurate.

In some alternative implementations of the above embodiments described with reference to Fig. 2, Fig. 4, and Fig. 5, the method for predicting a result of an appearance changing operation further includes: generating operating risk prompt information of the intended appearance changing operation of the target user based on a predicting result of the result predicting model of the appearance changing operation on the intended appearance changing operation of the target user.

In the present embodiment, the risk prompt information of the intended appearance changing operation of the target user is generated based on the predicting result of the appearance changing operation of the target user outputted from the result predicting model of the appearance changing operation. The predicting result may be a facial image and/or physical sign data of the target user after the appearance changing operation. The risk prompt information may be an appearance change degree generated based on comparison of the facial image before the appearance changing operation with the facial image after the appearance changing operation, or a physical sign data report generated based on the physical sign data after the appearance changing operation. For example, the appearance changing operation is a double eyelid operation item in a medical beauty surgery. If the predicting result of the appearance changing operation of the target user outputted from the result predicting model of the appearance changing operation is that eyelid changes in the facial image are not obvious and the target user will suffer from a sequelae, e.g., ceratitis, a surgical risk prompt is sent to the target user based on the predicting result. In the present embodiment, risk prompt information is sent to the target user based on the predicting result, thereby further helping the target user to make a decision on whether to perform the appearance changing operation.

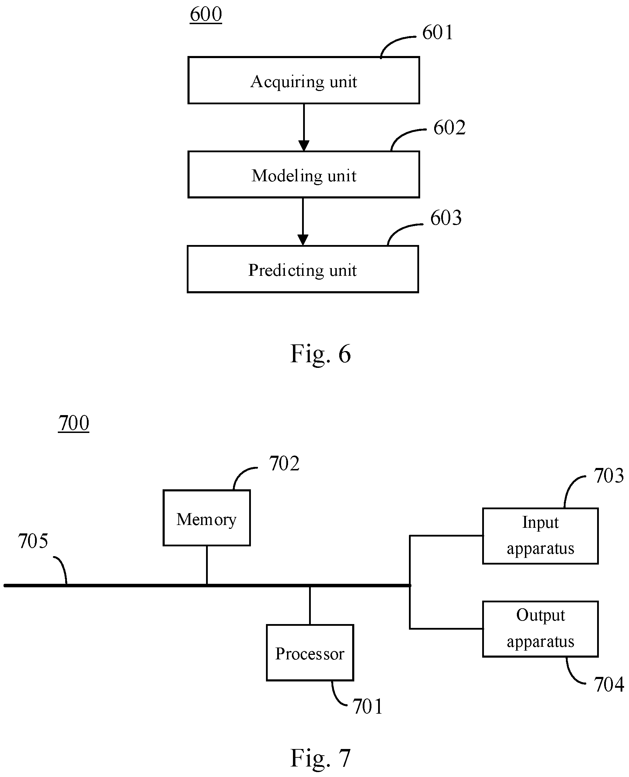

Further referring to Fig. 6, as an implementation of the method shown in the above figures, an embodiment of the present disclosure provides an apparatus for predicting a result of an appearance changing operation. The embodiment of the apparatus corresponds to the embodiment of the method shown in Fig. 2. The apparatus may be specifically applied to various electronic devices.

As shown in Fig. 6, the present embodiment provides an apparatus 600 for predicting a result of an appearance changing operation. The apparatus 600 includes: an acquiring unit 601, a modeling unit 602, and a predicting unit 603. The acquiring unit 601 is configured to acquire sample data of the appearance changing operation, the sample data including first appearance data of a user before the appearance changing operation and second appearance data after the appearance changing operation corresponding to the first appearance data; the modeling unit 602 is configured to establish a result predicting model of the appearance changing operation based on the first appearance data and the second appearance data; and the predicting unit 603 is configured to acquire appearance data and an intended appearance changing operation of a target user, to predict appearance data of the target user after the intended appearance changing operation using the result predicting model of the appearance changing operation based on the appearance data and the intended appearance changing operation of the target user.

In some embodiments, the modeling unit includes: an extracting module configured to perform key point feature extraction on the first appearance data and the second appearance data; and a first modeling module configured to model for a conversion relationship for converting a key point feature of the first appearance data to a key point feature of the second appearance data, to obtain the result predicting model.

In some embodiments, the apparatus further includes: acquiring physical state data of a user corresponding to the second appearance data; the modeling unit includes: a first modeling subunit configured to establish the result predicting model of the appearance changing operation based on the first appearance data and the corresponding second appearance data, and the physical state data of the user; and the apparatus further includes: a second modeling module configured to predict physical state information of the target user after the intended appearance changing operation using the result predicting model of the appearance changing operation.

In some embodiments, the sample data further includes first physical sign data of the user before the appearance changing operation and second physical sign data after the appearance changing operation corresponding to the first appearance data.

In some embodiments, the apparatus further includes: a third modeling module configured to establish a physical sign predicting model based on the first physical sign data and the corresponding second physical sign data; and a physical sign predicting module configured to acquire physical sign data and the intended appearance changing operation of the target user, to predict physical sign data of the target user after the intended appearance changing operation using the physical sign predicting model based on the physical sign data of the target user before the appearance changing operation.

In some embodiments, the modeling unit includes: a second modeling subunit configured to establish the result predicting model of the appearance changing operation based on the first appearance data and the corresponding second appearance data, the first physical sign data and the corresponding second physical sign data; and the apparatus further includes: a fourth modeling module configured to acquire physical sign data and the intended appearance changing operation of the target user, to predict physical sign data of the target user after the intended appearance changing operation using the result predicting model of the appearance changing operation based on the physical sign data and the intended appearance changing operation of the target user.

In some embodiments, the apparatus further includes: a prompting unit configured to generate operating risk prompt information of the intended appearance changing operation of the target user based on a predicting result of the result predicting model of the appearance changing operation on the intended appearance changing operation of the target user.

The units in the apparatus 600 may correspond to the steps in the method described in Fig. 2, Fig. 4, and Fig. 5. Therefore, the operations, features, and achievable technical effects described above for the method for predicting a result of an appearance changing operation also apply to the apparatus 600 and the units included therein. The description will not be repeated here.

According to an embodiment of the present disclosure, the present disclosure further provides an electronic device and a readable storage medium.

As shown in Fig. 7, a block diagram of an electronic device 700 of the method for predicting a result of an appearance changing operation according to embodiments of the present disclosure is shown. The electronic device is intended to represent various forms of digital computers, such as a laptop computer, a desktop computer, a workstation, a personal digital assistant, a server, a blade server, a mainframe computer, and other suitable computers. The electronic device may also represent various forms of mobile apparatuses, such as a personal digital assistant, a cellular phone, a smart phone, a wearable device, and other similar computing apparatuses. The components shown herein, the connections and relationships thereof, and the functions thereof are used as examples only, and are not intended to limit implementations of the present disclosure described and/or claimed herein.

As shown in Fig. 7, the electronic device includes: one or more processors 701, a memory 702, and interfaces for connecting various components, including a high-speed interface and a low-speed interface. The various components are interconnected using different buses, and may be mounted on a common motherboard or in other manners as required. The processor can process instructions for execution within the electronic device, including instructions stored in the memory or on the memory to display graphical information for a GUI on an external input/output apparatus (e.g., a display device coupled to an interface) . In other implementations, a plurality of processors and/or a plurality of buses may be used, as appropriate, along with a plurality of memories. Similarly, a plurality of electronic devices may be connected, with each device providing portions of necessary operations (e.g., as a server array, a group of blade servers, or a multi-processor system). In Fig. 7, a processor 701 is taken as an example.

The memory 702 is a non-transitory computer readable storage medium provided in embodiments of the present disclosure. The memory stores instructions executable by at least one processor, causing the at least one processor to perform the method for predicting a result of an appearance changing operation provided in embodiments of the present disclosure. The non-transitory computer readable storage medium of embodiments of the present disclosure stores computer instructions. The computer instructions are used for causing a computer to perform the method for predicting a result of an appearance changing operation provided in embodiments of the present disclosure.

As a non-transitory computer readable storage medium, the memory 702 may be configured to store non-transitory software programs, non-transitory computer-executable programs and modules, such as the program instructions/modules (e.g., the acquiring unit 601, the modeling unit 602, and the predicting unit 603 shown in Fig. 6) corresponding to the method for predicting a result of an appearance changing operation in embodiments of the present disclosure. The processor 701 runs non-transitory software programs, instructions, and modules stored in the memory 702, so as to execute various function applications and data processing of a server, i.e., implementing the method for predicting a result of an appearance changing operation in embodiments of the method.

The memory 702 may include a program storage area and a data storage area, where the program storage area may store an operating system and an application program required by at least one function; and the data storage area may store, e.g., data created based on use of the electronic device for predicting a result of an appearance changing operation. In addition, the memory 702 may include a high-speed random-access memory, and may further include a non-transitory memory, such as at least one magnetic disk storage component, a flash memory component, or other non-transitory solid state storage components. In some embodiments, the memory 702 alternatively includes memories disposed remotely relative to the processor 701, and these remote memories may be connected to the electronic device for predicting a result of an appearance changing operation via a network. Examples of the above network include, but are not limited to, the Internet, an intranet, a local area network, a mobile communication network, and a combination thereof.

The electronic device of the method for predicting a result of an appearance changing operation may further include: an input apparatus 703, an output apparatus 704, and a bus 707. The processor 701, the memory 702, the input apparatus 703, and the output apparatus 704 may be connected through a bus 705 or in other manners . A connection through the bus 705 is taken as an example in Fig. 7.

The input apparatus 703 may receive input digital or character information, and generate key signal inputs related to user settings and function control of the electronic device for performing the method for predicting a result of an appearance changing operation, such as touch screen, keypad, mouse, trackpad, touchpad, pointing stick, one or more mouse buttons, trackball, joystick and other input apparatuses. The output apparatus 704 may include a display device, an auxiliary lighting apparatus (for example, LED), a tactile feedback apparatus (for example, a vibration motor), and the like. The display device may include, but is not limited to, a liquid crystal display (LCD), a light emitting diode (LED) display, and a plasma display. In some embodiments, the display device may be a touch screen.

Various implementations of the systems and techniques described herein may be implemented in a digital electronic circuit system, an integrated circuit system, an application specific integrated circuit (ASIC), computer hardware, firmware, software, and/or combinations thereof. These various implementations may include the implementation in one or more computer programs. The one or more computer programs may be executed and/or interpreted on a programmable system including at least one programmable processor, and the programmable processor may be a dedicated or general-purpose programmable processor, may receive data and instructions from a storage system, at least one input apparatus and at least one output apparatus, and transmit the data and the instructions to the storage system, the at least one input apparatus and the at least one output apparatus.

These computing programs, also referred to as programs, software, software applications or codes, include a machine instruction of the programmable processor, and may be implemented using a high-level procedural and/or an object-oriented programming language, and/or an assembly/machine language. As used herein, the terms "machine readable medium" and "computer readable medium" refer to any computer program product, device and/or apparatus (e.g., a magnetic disk, an optical disk, a storage device and a programmable logic device (PLD)) used to provide a machine instruction and/or data to the programmable processor, and include a machine readable medium that receives the machine instruction as a machine readable signal. The term "machine readable signal" refers to any signal used to provide the machine instruction and/or data to the programmable processor.

To provide an interaction with a user, the systems and techniques described here may be implemented on a computer having a display apparatus (e.g., a cathode ray tube (CRT)) or an LCD monitor) for displaying information to the user, and a keyboard and a pointing apparatus (e.g., a mouse or a track ball) by which the user may provide the input to the computer. Other kinds of apparatuses may also be used to provide the interaction with the user. For example, a feedback provided to the user may be any form of sensory feedback (e.g., a visual feedback, an auditory feedback, or a tactile feedback) ; and an input from the user may be received in any form, including acoustic, speech, or tactile input.

The systems and techniques described here may be implemented in a computing system (e.g., as a data server) that includes a backend part, implemented in a computing system (e.g., an application server) that includes a middleware part, implemented in a computing system (e.g., a user computer having a graphical user interface or a Web browser through which the user may interact with an implementation of the systems and techniques described here) that includes a frontend part, or implemented in a computing system that includes any combination of the backend part, the middleware part or the frontend part. The parts of the system may be interconnected by any form or medium of digital data communication (e.g., a communication network). Examples of the communication network include a local area network (LAN), a wide area network (WAN) and Internet.

The computer system may include a client and a server. The client and the server are generally remote from each other and typically interact through the communication network. The relationship between the client and the server is generated through computer programs running on the respective computers and having a client-server relationship to each other.

According to an embodiment of the present disclosure, the present disclosure further provides a computer program product including a computer program, where the computer program, when executed by a processor, implements the method for predicting a result of an appearance changing operation.

It should be understood that the various forms of processes shown above can be used to reorder, add, or delete steps. For example, the steps disclosed in embodiments of the present disclosure can be executed in parallel, sequentially, or in different orders, as long as the desired results of the technical solutions disclosed in embodiments of the present disclosure can be achieved. This is not limited herein.

The above specific implementations do not constitute a limitation to the protection scope of the present disclosure. It should be understood by those skilled in the art that various modifications, combinations, sub-combinations, and replacements may be made according to the design requirements and other factors. Any modification, equivalent replacement, improvement, and the like made within the spirit and principle of the present disclosure should be included within the protection scope of the present disclosure.

## Claims

1. A method for predicting a result of an appearance changing operation, comprising:
acquiring (201) sample data of the appearance changing operation, the sample data comprising first appearance data of a user before the appearance changing operation and second appearance data after the appearance changing operation corresponding to the first appearance data;
establishing (202) a result predicting model of the appearance changing operation based on the first appearance data and the second appearance data; and
acquiring (203) appearance data and an intended appearance changing operation of a target user, to predict appearance data of the target user after the intended appearance changing operation using the result predicting model of the appearance changing operation based on the appearance data and the intended appearance changing operation of the target user.

2. The method according to claim 1, wherein the establishing (202) the result predicting model of the appearance changing operation based on the first appearance data and the second appearance data comprises:
performing key point feature extraction on the first appearance data and the second appearance data; and
modeling a conversion relationship for converting a key point feature of the first appearance data to a key point feature of the second appearance data, to obtain the result predicting model.

3. The method according to claim 1 or 2, wherein the method further comprises: acquiring physical state data of the user corresponding to the second appearance data; and
the establishing (202) the result predicting model of the appearance changing operation based on the first appearance data and the second appearance data comprises:
establishing the result predicting model of the appearance changing operation based on the first appearance data and the corresponding second appearance data, and the physical state data of the user; and
the method further comprises:
predicting physical state information of the target user after the intended appearance changing operation using the result predicting model of the appearance changing operation.

4. The method according to any one of claims 1-3, wherein the sample data further comprises first physical sign data of the user before the appearance changing operation and second physical sign data after the appearance changing operation corresponding to the first appearance data.

5. The method according to claim 4, wherein the method further comprises:
establishing (402) a physical sign predicting model based on the first physical sign data and the corresponding second physical sign data; and
acquiring (403) physical sign data and the intended appearance changing operation of the target user, to predict physical sign data of the target user after the intended appearance changing operation using the physical sign predicting model based on the physical sign data of the target user.

6. The method according to claim 4, wherein the establishing (202) the result predicting model of the appearance changing operation based on the first appearance data and the second appearance data comprises:
establishing (502) the result predicting model of the appearance changing operation based on the first appearance data and the corresponding second appearance data, the first physical sign data and the corresponding second physical sign data; and
the method further comprises:
acquiring (503) physical sign data and the intended appearance changing operation of the target user, to predict physical sign data of the target user after the intended appearance changing operation using the result predicting model of the appearance changing operation based on the physical sign data and the intended appearance changing operation of the target user.

7. The method according to any one of claims 1-6, wherein the method further comprises:
generating operating risk prompt information of the intended appearance changing operation of the target user based on a predicting result of the result predicting model of the appearance changing operation on the intended appearance changing operation of the target user.

8. An apparatus for predicting a result of an appearance changing operation, comprising a plurality of modules (601, 602, 603) configured to implement the method according to any one of claims 1-7.

9. An electronic device, comprising:
at least one processor (701); and
a memory (702) communicatively connected with the at least one processor (701);
the memory (702) storing instructions executable by the at least one processor (701), and the instructions, when executed by the at least one processor (701), causing the at least one processor (701) to perform the method according to any one of claims 1-7.

10. A non-transitory computer readable storage medium (702) storing computer instructions, the computer instructions being used for causing a computer to perform the method according to any one of claims 1-7.

11. A computer program product comprising a computer program, the computer program, when executed by a processor (701), implementing the method according to any one of claims 1-7.
